# EUROPEAN PATENT SPECIFICATION

(11) **EP 0 851 852 B1**
(45) Date of publication and mention of the grant of the patent: **05.04.2000**
(21) Application number: 96926472.0
(22) Date of filing: 06.08.1996
(51) Int. Cl.: C07C 201/08, C07C 205/59, C07C 303/40

(54) **PROCESS FOR NITRATING DIPHENYL ETHER COMPOUNDS**
VERFAHREN ZUR NITRIERUNG VON DIPHENYL ETHER VERBINDUNGEN
PROCEDE DE NITRATION DE COMPOSES D'ETHER DIPHENYLIQUE

(30) Priority: 13.09.1995 GB 9518705
(43) Date of publication of application: 08.07.1998
(73) Proprietor: ZENECA LIMITED, London W1Y 6LN (GB)
(72) Inventor: BROWN, Stephen, Martin, West Yorkshire HD8 8NX (GB); MUXWORTHY, James, Peter, Wakefield West Yorkshire WF4 4RB (GB)
(74) Representative: Waterman, John Richard
(86) International application number: GB9601892
(87) International publication number: WO9710199

(56) References cited:
- EP-A- 0 147 798
- EP-A- 0 668 260
- GB-A- 2 103 214
- US-A- 4 429 146
- US-A- 4 743 703

## Description

The present invention relates to a process for nitration and, in particular to a process for nitrating diphenyl ether compounds which are useful as herbicides or as intermediates in the synthesis of herbicides.

EP-A-0022610 relates to herbicides of the formula: 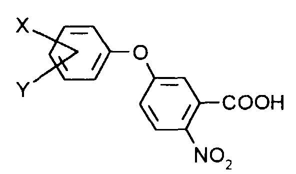 wherein X and Y may be H, F, Cl, Br, CF₃, OCF₂CHZ₂ (Z = Cl, Br, F), OCH₃, CN, CO₂R (R = lower alkyl), C₆H₅, O-alkyl, NO₂ or SO₂ lower alkyl;
and also describes a process for making these compounds by nitrating a compound of the formula: 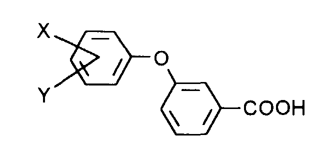 wherein X and Y are as defined above.

Suggested nitrating agents for this reaction include mixtures of nitric and sulphuric acids and the recommended reaction solvent is dichloromethane. The nitration process is said to give a yield of 75.4% but no details are given of the purity of the product or the presence of other nitrated isomers.

US 4,031,131 describes similar compounds to the above which are prepared in a similar manner. Suggested nitrating agents include potassium nitrate or mixed nitric and sulphuric acids and the reaction is carried out in dichloromethane. An extremely high yield (>95%) is claimed for the nitration reaction but, again, there are no details given about the purity of the product. Nitration reactions using mixed nitric and sulphuric acids may also be carried out in the presence of acetic anhydride.

EP-A-0003416 and EP-A-0274194 both relate to the synthesis of herbicidal compounds of the formula: 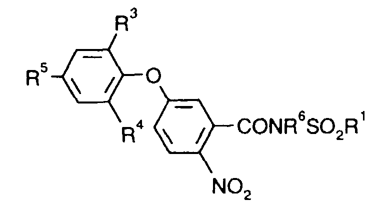 wherein
R¹ is alkyl optionally substituted with fluorine or optionally substituted phenyl;
R³ is H, F, Cl, Br, I alkyl, trifluoromethyl or CN;
R⁴ is H, F, Cl, Br, I or trifluoromethyl;
R⁵ is F, Cl, Br, I or trifluoromethyl; and R⁶ is H or C₁-C₄ alkyl.

In EP-A-0003416, these compounds may be obtained by nitrating the corresponding carboxylic acid or carboxamide and then converting to the sulphonamide or by nitrating the sulphonamide itself. A nitration reaction is described in Example 7 where the solvent is 1,2-dichloroethane and the nitrating agent is a mixture of potassium nitrate and concentrated sulphuric acid.

The nitration of substituted diphenyl ethers is also discussed in US 4,743703 and US 4,429,146 and the art cited in these documents

EP-A-0274194 relates, in particular, to a process for the nitration of compounds of the formula:

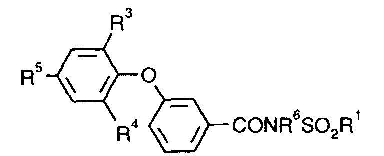

The nitration reaction is said to be carried out using a conventional nitrating agent such as concentrated nitric acid or sodium nitrate or mixtures of these with sulphuric acid. The reaction solvent is one which is resistant to nitration and examples of such solvents are said to include halogenated solvents such as dichloromethane, dichloroethane, dichloropropane, chlorofluorocarbons and aromatic solvents such as nitrobenzene.

However, none of these methods are particularly satisfactory for use on an industrial scale because they all have the common problem that the reaction yields a mixture of the required product and other nitrated isomers. Nitrated isomers of diphenyl ether compounds are often extremely difficult to separate from one another and the quantity of other isomers is often too high for the final product to fulfil the requirements of the regulatory authorities for herbicides. The problem tends to be further exacerbated if the nitrated product is an intermediate in the synthesis of a herbicide rather than the required herbicide itself, because the mixture of nitrated compounds means that larger quantities of other reagents must be used than would be necessary if the nitrated isomers could be separated satisfactorily. It is therefore important to ensure that the nitration process produces a product mixture containing the highest possible proportion of the desired isomer.

The problem of obtaining mixtures of isomers from the nitration process was recognised by the authors of GB-A-2103214 who describe a process in which a compound of the formula: 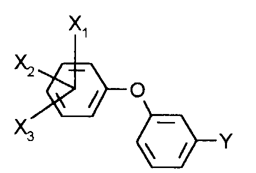 wherein
each of X₁, X₂ and X₃ is H, fluorine, chlorine, bromine, CF₃, O CF₂,CHZ₂ ( where Z is F, Cl or Br), OCF₃, CN, COOR (R is lower alkyl), phenyl, lower alkoxy or NO₂R and at least one of X₁, X₂ and X₃ is other than hydrogen, and
Y is COOR or carboxy;
is nitrated to give a product of the formula: 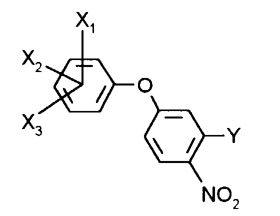 wherein X₁, X₂, X₃ and Y are as defined above.

The nitration is carried out using as nitrating agent a mixture of nitric and sulphuric acids in an organic solvent such as dichloromethane. The desirability of keeping the reaction system anhydrous by the addition of acetic anhydride is stressed as the authors of GB-A-2103214 state that this makes it possible to improve the selectivity with respect to Acifluorfen (the desired nitrated product). The recommended ratio of starting material : solvent : acetic anhydride is 1:2.66:1.4. The reaction is conducted at a temperature of 45°C and left for 3 hours. After this, the reaction mixture is allowed to stand so that the organic and aqueous phases separate and then the organic solvent is removed by distillation.

However, the present inventors have found that the use of reaction conditions suggested lead to various problems which do not seem to have been appreciated by the authors of the prior art document. In particular, although the use of acetic anhydride does, in some respects, improve the selectivity of the reaction, the relationship between the concentration of acetic anhydride and selectivity is more complex than the authors of GB-A-2103214 appear to have realised and, therefore, the amount of acetic anhydride in the reaction mixture must be carefully controlled in order to obtain a suitable product mixture.

Therefore in the present invention there is provided a process for the preparation of a compound of general formula I: 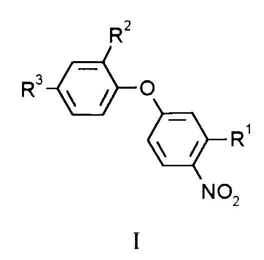 wherein:
R¹ is hydrogen or C₁-C₆ alkyl, C₂-C₆ alkenyl or C₂-C₆ alkynyl (any of which may optionally be substituted with one or more substituents selected from halogen and OH) or COOR⁴, COR⁶, CONR⁴R⁵ or CONHSO₂R⁴;
   R⁴ and R⁵ are each independently hydrogen or C₁-C₄ alkyl optionally substituted with one or more halogen atoms;
   R⁶ is a halogen atom or a group R⁴;
R² is hydrogen or halo;
R³ is C₁-C₄ alkyl, C₂-C₄ alkenyl or C₂-C₄ alkynyl (any of which may optionally be substituted with one or more halogen atoms) or halo;
the process comprising reacting a compound of general formula II: 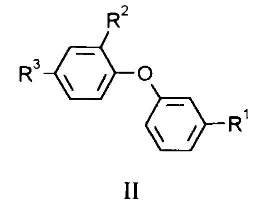 wherein R¹, R² and R³ are as defined for general formula I;
with a nitrating agent comprising nitric acid or a mixture of nitric and sulphuric acids in the presence of an organic solvent and in the presence of acetic anhydride, wherein the molar ratio of acetic anhydride to compound of general formula II is from about 1:1 to 3:1, characterised in that the organic solvent is tetrachloroethylene (perklone).

These reaction conditions give the advantage that the proportion of the required isomer is maximised whilst not causing too great a reduction in the yield of the product or too great an increase in operating costs.

In the context of the present invention, compounds of general formula I are designated 4-nitro isomers. The 2-nitro isomers referred to above have the general formula: 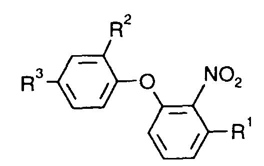 Other mono-nitro isomers which may be produced in the nitration reaction include the 6-nitro isomer:

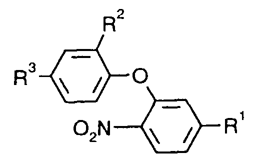

There are also three different dinitro isomers which may be present.

In the context of the present invention, the term "C₁-C₆ alkyl" refers to a saturated straight or branched hydrocarbon chain containing from 1 to 6 carbon atoms. Examples include methyl, ethyl, n-propyl, t-butyl, n-pentyl and n-hexyl. The term "C₁-C₄ alkyl" is a subset of C₁-C₆ alkyl and refers to an alkyl group having up to 4 carbon atoms.

The term "C₂-C₆ alkenyl" refers to a straight or branched hydrocarbon chain containing from 2 to 6 carbon atoms and having at least one double bond. Examples include ethenyl, allyl, propenyl and hexenyl. The term "C₂-C₄ alkenyl" is a subset of C₂-C₆ alkenyl and refers to an alkenyl group having up to 4 carbon atoms.

The term "C₂-C₆ alkynyl" refers to a straight or branched hydrocarbon chain containing from 2 to 6 carbon atoms and having at least one triple bond. Examples include ethynyl, propynyl and hexynyl. The term "C₂-C₄ alkynyl" is a subset of C₂-C₆ alkynyl and refers to an alkynyl group having up to 4 carbon atoms.

The term "halogen" refers to fluorine, chlorine, bromine or iodine and the corresponding term "halo" refers to fluoro, chloro, bromo or iodo.

The reaction conditions of the present invention are particularly advantageous since they maximise the amount of the required 4-nitro isomer in the product mixture. Surprisingly, it has been found by the present inventors that the relationship between the presence of acetic anhydride and the isomer ratio of the product mixture is not as simple as it appears from a reading of GB-A-2103214. This document suggests that the presence of acetic anhydride is beneficial but does not suggest that the amount present needs to be limited. The present inventors have found, however, that although the proportion of dinitro isomers (1) and (2) in the product mixture decreases as the amount of acetic anhydride is increased, the proportion of the 2-nitro impurity increases. This is a particular concern since the 2-nitro isomer is especially difficult to separate from the 4-nitro isomer and so, clearly, it is important to keep its concentration in the product mixture as low as possible. For this reason, the present inventors have found that it is not desirable to increase the acetic anhydride : compound II ratio to greater than about 3:1.

Additionally, the present inventors have discovered that the reaction temperature plays a significant role in determining the proportions of the various mono-nitrated isomers with a greater proportion of the required isomer being produced as the reaction temperature is reduced. The reaction temperature, too is a compromise since, clearly, it would not be economically viable to operate a reaction if the temperature were below a certain level because of the amount of cooling required. The decrease with temperature of the proportion of the 2-nitro and 6-nitro isomers in the product mixture does not seem to have been appreciated by the authors of GB-A-2103214 who recommended a reaction temperature of about 45°C. The present inventors have found that the amount of the 2-nitro isomer present in the product mixture when the reaction temperature is 45°C is more than 12 parts per hundred whereas, when the reaction temperature is reduced to 10°C, the amount of 2-nitro isomer in the product mixture is reduced to 10 or 11 parts per hundred. This difference may affect any subsequent purification process and may be very significant when costing a large scale manufacturing process. The preferred temperature range for the process of the present invention is from about -15° to 15°C , more preferably -10° to 10°C.

It has also been found that the formation of the undesired isomers can be further reduced by increasing the concentration of the reactants in the solvent solution. In particular, it is advantageous to have a weight ratio of solvent to reactant (including any isomers present) of no greater than 4.25:1 and it is preferred that the ratio is from 1:1 to 2.5:1.

The reaction is carried out in tetrachloroethylene (perklone).

Perklone is a particularly useful solvent for the process of the present invention since, under equivalent reaction conditions, Perklone reactions give about 30% less of the 2- and 6-nitro isomers than reactions carried out in EDC or DCM under otherwise identical conditions. There are also indications that the yield of the reaction is increased when Perklone is used as the solvent.

As already mentioned, the nitrating agent used is nitric acid or a mixture of nitric and sulphuric acids. A mixture of nitric and sulphuric acids may contain, for example, from about 30 to 45% of pure nitric acid, more typically from about 30 to 35% pure nitric acid.

When the chosen nitrating agent is a mixed acid, it will typically be added to the reaction mixture over a period of about 30 minutes to 15 hours.

It is often advantageous to add the nitrating agent over a period of from 5 to 15 hours, or, more preferably, 6 to 12 hours.
Although the process of the invention may be used for the preparation of any compound of general formula I, it is especially preferred that R² is chloro and R³ is trifluoromethyl. Particularly preferred compounds of general formula I are those in which R¹ is COOH or CONHSO₂CH₃. These compounds are 5-(2-chloro-α,α,α-trifluoro-4-tolyloxy)-2-nitrobenzoic acid (Acifluorfen) and 5-(2-chloro-α,α,α-trifluoro-4-tolyloxy)-N-methanesulphonyl-2-nitrobenzamide (Fomesafen), both of which are potent herbicides.

In addition to being a herbicide in its own right, Acifluorfen may also serve as an intermediate in the synthesis of Fomesafen. The Acifluorfen may be converted to the acid chloride which may then be reacted with methane sulphonamide to give Fomesafen. Both of these steps may be carried out by conventional methods, for example as set out in EP-A-0003416.

The invention will now be further described by way of the following examples in which the following abbreviations are used:
DCM - dichloromethane;
EDC - ethylene dichloride
pph - parts per hundred;
HPLC - high performance liquid chromatography.

In the examples, the term "mixed acid" refers to a mixture containing 33.6% nitric acid and 66.4% sulphuric acid. The molar quantities given are the moles of nitric acid in the mixture.

### COMAPARATIVE EXAMPLE A

### General method for nitration of 3-(2-chloro-α,α,α-trifluoro-4-tolyloxy)benzoic acid in dichloromethane to yield Acifluorfen

### Nitration

Acetic anhydride (see Tables I and II for amounts) was added to 3-(2-chloro-α,α,α-trifluoro-4-tolyloxy)benzoic acid (I, R¹ is COOH, R² is chloro, R³ is trifluoromethyl) (20g, 0.063 mol) in dichloromethane (54g, 0.635 mol) and the mixture stirred and heated to 40°C to dissolve the starting material. The mixture was then cooled to the appropriate reaction temperature (during which time any crystallisation of the starting material was observed). Mixed acid (13g, 0.069 mol) was added dropwise over a period of 2 hours and the reaction monitored by HPLC for the completion of the reaction. Further additions of Mixed acid were made to reduce the level of starting material to about 1 pph.

### Work-Up

The reaction mixture was washed three times as follows:
wash 1 - water (30ml) was added and the mixture washed at approximately 38°C and the aqueous layer separated;
wash 2 - water (25ml) was added and the mixture washed at approximately 38°C and the aqueous layer separated;
wash 3 - water (25ml) was added and the mixture washed at approximately 38°C and the aqueous layer separated.

Water (80ml) was then added and the mixture heated to 38°C and sodium hydroxide (47% solution, 6.4g, 0.076 mol) added to basify the mixture to pH 10-11. The mixture was heated to distil off the DCM in order to afford a solution of Acifluorfen sodium salt. The solution was cooled to room temperature and transferred with the aid of a minimum amount of water to a bottle in order for the solution to be weighed and analysed.

The results for various amounts of acetic anhydride and various reaction temperatures are shown in Table I (see Experiments 1 to 11).

### COMPARATIVE EXAMPLE B

### General method for nitration of 3-(2-chloro-α,α,α-trifluoro-4-tolyloxy)benzoic acid in ethylene dichloride to yield Acifluorfen

### Nitration

Acetic anhydride (see Tables I and II for amounts) was added to 3-(2-chloro-α,α,α-trifluoro-4-tolyloxy)benzoic acid (20g, 0.063 mol) in ethylene dichloride (54g, 0.545 mol) and the mixture stirred and heated to 40°C to dissolve the starting material. The mixture was then cooled to the appropriate reaction temperature (during which time any crystallisation of the starting material was observed). Mixed acid (33.6%, 13g, 0.069 mol) was added dropwise over a period of 2 hours and the reaction monitored by HPLC for the completion of the reaction. Further additions of Mixed acid were made to reduce the level of starting material to about 1 pph.

### Work-Up

The reaction mixture was washed three times as follows:
wash 1 - water (30ml) was added and the mixture washed at approximately 70°C and the aqueous layer separated;
wash 2 - water (25ml) was added and the mixture washed at approximately 70°C and the aqueous layer separated;
wash 3 - water (25ml) was added and the mixture washed at approximately 70°C and the aqueous layer separated.

Water (80ml) was then added and the mixture heated to 80°C and sodium hydroxide (47% solution, 6.4g, 0.076 mol) added to basify the mixture to pH 10-11. The mixture was allowed to separate and the EDC layer was removed. Traces of residual EDC were then removed by distillation to afford a solution of Acifluorfen sodium salt. The solution was cooled to room temperature and transferred with the aid of a minimum amount of water to a bottle in order for the solution to be weighed and analysed.

### EXAMPLE 1

### General method for nitration of 3-(2-chloro-α,α,α-trifluoro-4-tolyloxy)benzoic acid in Perklone to yield Acifluorfen

The general method and quantities of reagents were exactly as described for Comparative Examples A and B except that the solvent used was Perklone.

The results for Experiments 1 to 45 which were conducted according to the general methods of the Examples are set out in the Tables below. In these experiments, the amounts of acetic anhydride, the reaction temperature, the solvent and the quantity of solvent were varied in order to determine the optimum reaction conditions. In each of these experiments, 20g crude starting material was used containing 84.3% 3-(2-chloro-α,α,α-trifluoro-4-tolyloxy)benzoic acid. In each of the experiments described in Table I, the amount of solvent used was 54.0g but for the experiments detailed in Table II, the quantity of solvent was varied. In Tables I and II, the term "reactant" refers to 3-(2-chloro-α,α,α-trifluoro-4-tolyloxy) benzoic acid and the following abbreviations are used:
Exp Experiment No.
pph Parts per hundred
Ac₂O Acetic anhydride;
DCM Dichloromethane
EDC Ethylene dichloride

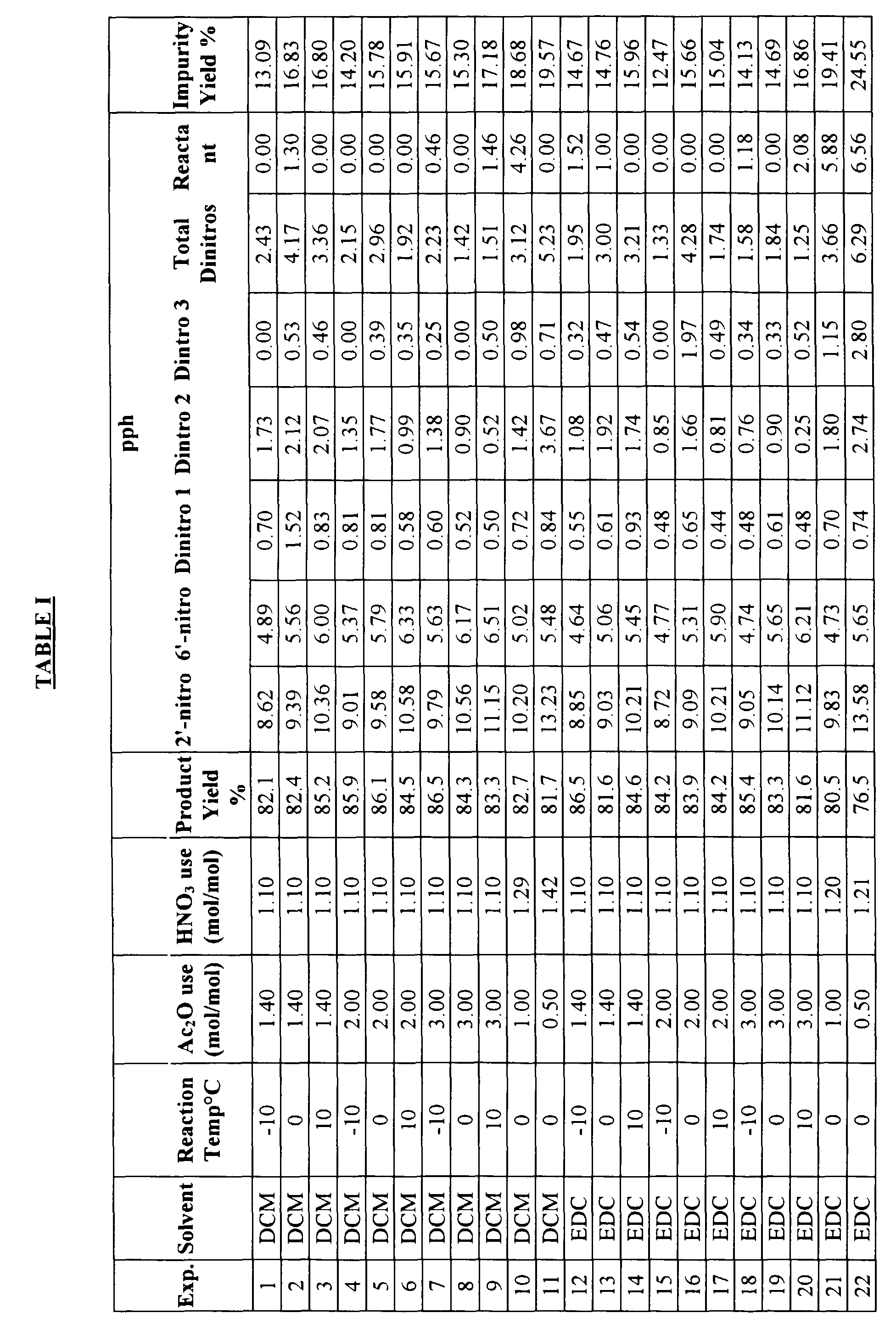

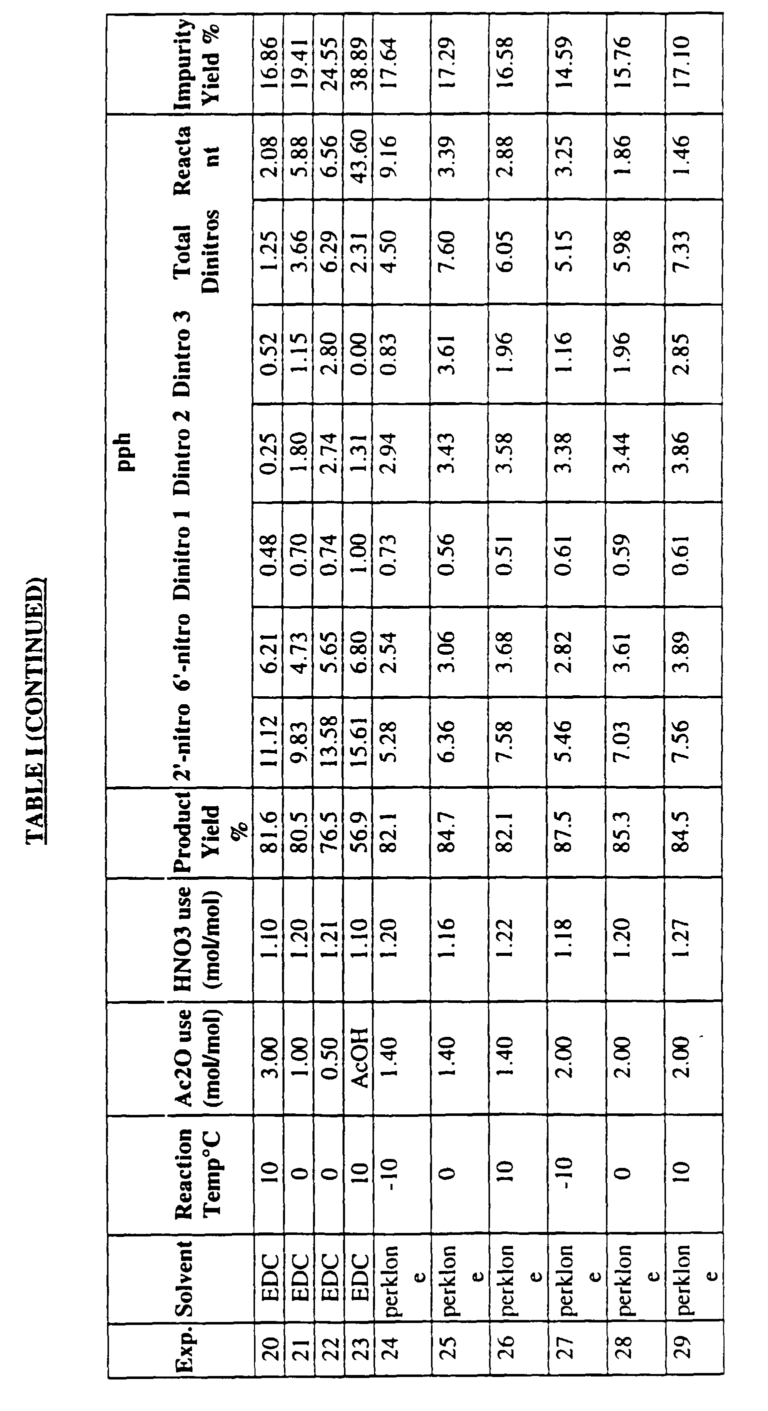

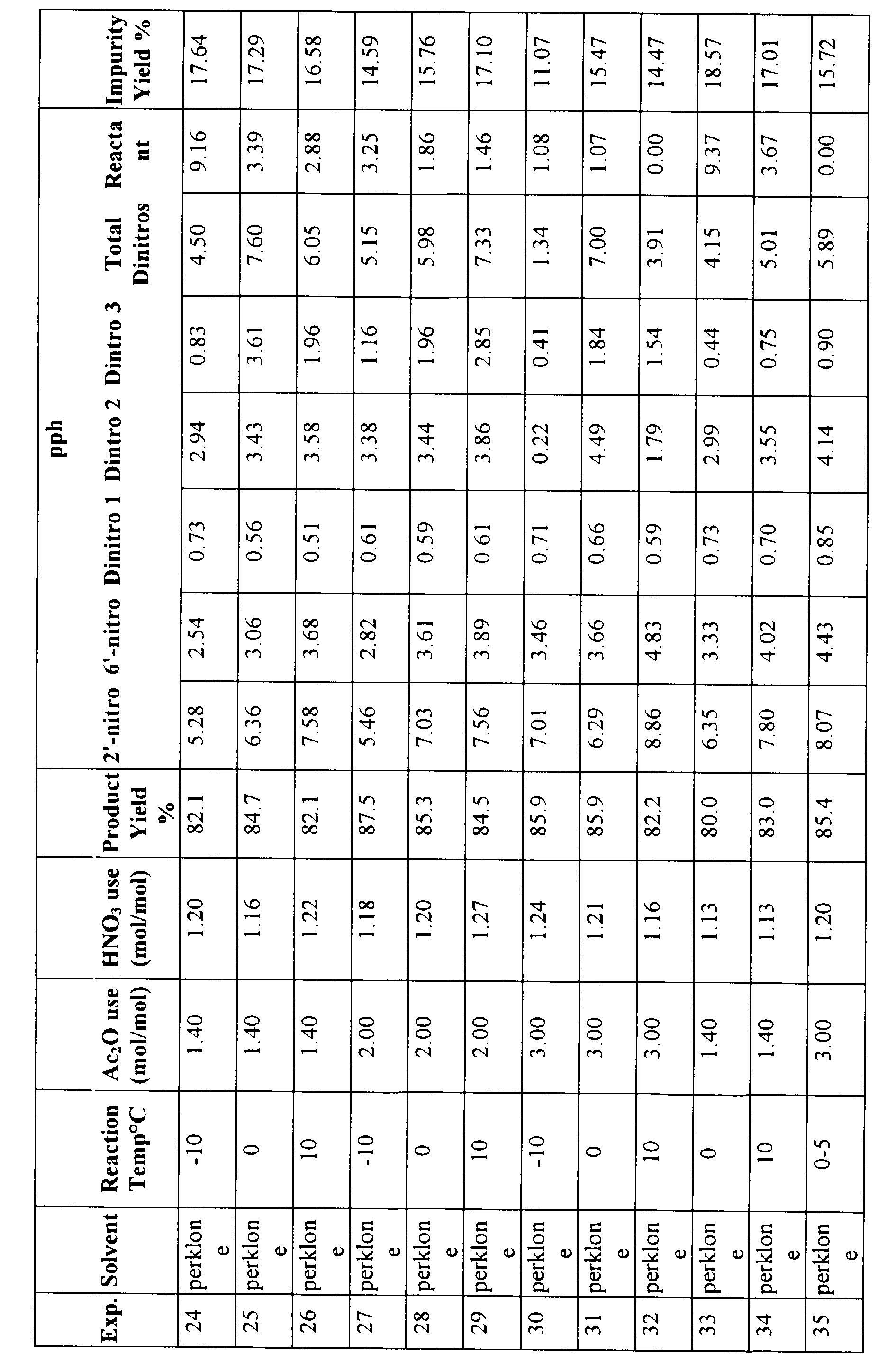

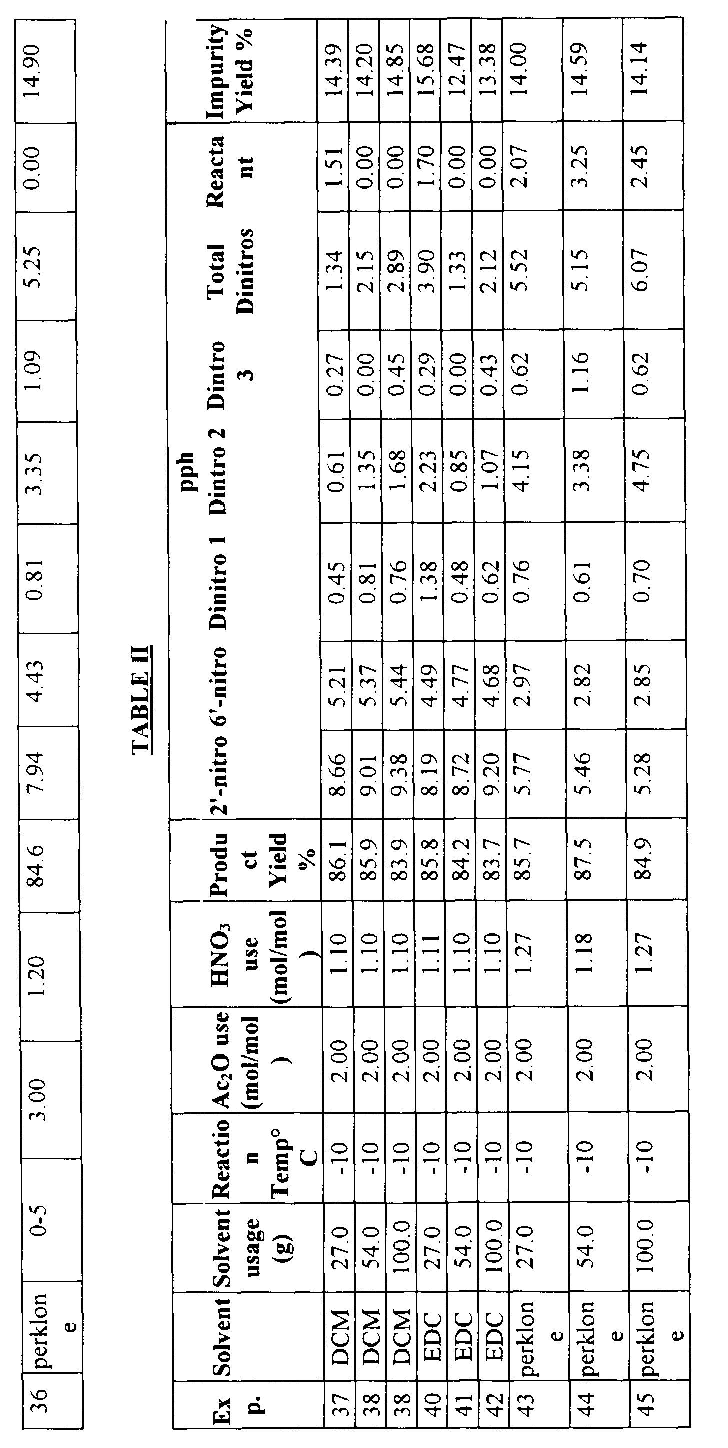

The results presented in Table I demonstrate the effects on the concentration of impurities in the final product of changing the molar ratio of acetic anhydride to starting material, temperature and the solvent.

Firstly, the effect of acetic anydride : starting material can be seen from a comparison of the results for Experiments 11, 10, 2, 5 and 8 of Table I, all of which were conducted using DCM as solvent and at a temperature of 0°C. The table shows that while the total concentration of dinitro impurities in the product mixture fell as the ratio of acetic anhydride : starting material increased, the amounts of the 2-nitro and 6-nitro isomers in the product mixture did not follow this pattern. Thus, for acetic anhydride ratios of 0.5, 1.0, 1.4, 2.0 and 3.0, the amounts of 2-nitro isomer present in the product mixture expressed in pph were 13.23, 10.2, 9.39, 9.58 and 10.56 whilst corresponding values for the 6-nitro isomer were 5.48, 5.02, 5.56, 5.79 and 6.17. Since the 2- and 6-nitro isomers are more difficult to separate from Acifluorfen than the dinitro isomers, it is obviously preferable to minimise the production of these mono nitro isomers and, thus, it can be seen that, for optimum performance, the molar ratio of acetic anhydride to starting material must be maintained at from about 1:1 to 3:1.

The effect of temperature can be seen by comparing, for example, the results of Experiments 1 to 3 or 12 to 14 or 24 to 26. It is clear that, in general, the amounts of all the impurities in the product mixture increase as the temperature increases.

Solvent effects are also apparent from Table I and it can be seen that, whilst the amounts of 2-nitro and 6-nitro impurities in the product mixtures are similar for DCM and EDC, they are about 32% lower when Perklone is used as the solvent. Perklone is the solvent used in the present invention.

The results of experiments to test the effect of varying the amount of solvent present in the reaction mixture are shown in Table II. From this table it can be seen that, in general, the amounts of 2-nitro and 6-nitro isomers present in the product mixuture increase as the reaction mixture becomes more dilute.

### EXAMPLE 2

### Nitration of 3-(2-chloro-α,α,α-trifluoro-4-tolyloxy)-N-(methylsulphonyl) benzamide in dichloromethane to yield Acifluorfen

3-(2-chloro-α,α,α-trifluoro-4-tolyloxy)-N-(methylsulphonyl) benzamide (10.4g, 0.0264 mol) was dispersed in dichloromethane (25.9g) with stirring. Acetic anhydride (11.4g, 98%, 0.110 mol) was added to the mixture over about 30 minutes maintaining the temperature at about 20°C. Mixed nitric and sulphuric acids (32.6% nitric acid, 0.0317 mol) were added slowly over about 45 minutes, following which the reaction mixture was heated to about 40° to 45°C for 3 hours. The reaction mass was washed with water and the solvent was removed by distillation to give 10.4g, 85.2% yield of the required product, Fomesafen. The product mixture also contained 6.8 pph 2-nitro isomer and 5.3pph 6-nitro isomer.

## Claims

1. A process for the preparation of a compound of general formula I: 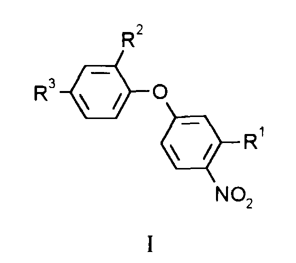 wherein:
R¹ is hydrogen or C₁-C₆ alkyl, C₂-C₆ alkenyl or C₂-C₆ alkynyl (any of which may optionally be substituted with one or more substituents selected from halogen and OH) or COOR⁴, COR⁶, CONR⁴R⁵ or CONHSO₂R⁴;
R⁴ and R⁵ are each independently hydrogen or C₁-C₄ alkyl optionally substituted with one or more halogen atoms;
R⁶ is a halogen atom or a group R⁴;
R² is hydrogen or halo;
R³ is C₁-C₄ alkyl, C₂-C₄ alkenyl or C₂-C₄ alkynyl (any of which may optionally be substituted with one or more halogen atoms) or halo;
the process comprising reacting a compound of general formula II: 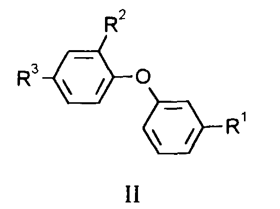 wherein R¹, R² and R³ are as defined for general formula I;
with a nitrating agent comprising nitric acid or a mixture of nitric and sulphuric acids in the presence of an organic solvent and in the presence of acetic anhydride, wherein the molar ratio of acetic anhydride to compound of general formula II is from about 1:1 to 3:1, characterised in that the organic solvent is tetrachloroethylene (perklone).

2. A process as claimed in claim 1, wherein the weight ratio of solvent to reactant (including any isomers present) is no greater than 4.25:1.

3. A process as claimed in claim 2, wherein the weight ratio of solvent to reactant (including any isomers present) is from 1:1 to 2.5:1.

4. A process as claimed in any one of claims 1 to 3, wherein the nitrating agent is a mixture of nitric and sulphuric acids containing from 30 to 45% of pure nitric acid.

5. A process as claimed in claim 4, wherein the nitrating agent is added to the reaction mixture over a period of about 30 minutes to 15 hours.

6. A process as claimed in any one of claims 1 to 5, wherein, in the compound of general formula I, R² is chloro and R³ is trifluoromethyl.

7. A process as claimed in claim 6 wherein the compound of general formula I is 5-(2-chloro-α,α,α-trifluoro-4-tolyloxy)-2-nitrobenzoic acid (Acifluorfen) or 5-(2-chloro-α,α,α-trifluoro-4-tolyloxy)-N-methanesulphonyl-2-nitrobenzamide (Fomesafen).

8. A process as claimed in claim 7 wherein the compound of general formula I is Acifluorfen and which further comprises the steps of converting the Acifluorfen to its acid chloride and treating the acid chloride with methane sulphonamide to give Fomesafen.

## Patentansprüche

1. Verfahren zur Herstellung einer Verbindung mit der allgemeinen Formel (I): 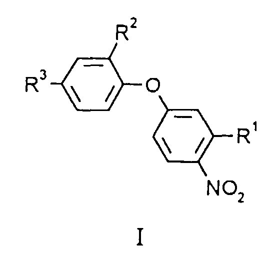 worin:
R¹ Wasserstoff oder C₁₋₆-Alkyl, C₂₋₆-Alkenyl oder C₂₋₆-Alkinyl (von denen jedes gegebenenfalls mit einem oder mehreren Substituenten substituiert sein kann, ausgewählt aus Halogen und OH) oder COOR⁴, COR⁶, CONR⁴R⁵ oder CONHSO₂R⁴ ist;
R⁴ und R⁵ jeweils unabhängig Wasserstoff oder C₁₋₄-Alkyl sind, gegebenenfalls mit einem oder mehreren Halogenatomen substituiert;
R⁶ ein Halogenatom oder eine Gruppe R⁴ ist;
R² Wasserstoff oder Halogen ist;
R³ C₁₋₄-Alkyl, C₂₋₄-Alkenyl oder C₂₋₄-Alkinyl (von denen jedes gegebenenfalls mit einem oder mehreren Halogenatomen substituiert sein kann) oder Halogen ist;
wobei das Verfahren die Umsetzung einer Verbindung der allgemeinen Formel (II): 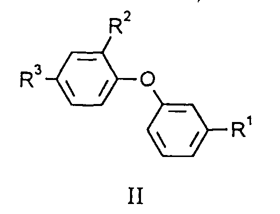 worin R¹, R² und R³ wie für die allgemeine Formel (I) definiert sind;
mit einem Nitrierungsmittel, umfassend Salpetersäure oder eine Mischung aus Salpeter- und Schwefelsäure, in Gegenwart eines organischen Lösungsmittels und in Gegenwart von Essigsäureanhydrid umfaßt, worin das Molverhältnis von Essigsäureanhydrid zur Verbindung der allgemeinen Formel (II) ca. 1:1 bis 3:1 beträgt, dadurch gekennzeichnet, daß das organische Lösungsmittel Tetrachlorethylen (Perklon) ist.

2. Verfahren gemäß Anspruch 1, worin das Gewichtsverhältnis von Lösungsmittel zu Reaktand (einschließlich aller vorhandenen Isomere) nicht mehr als 4,25:1 beträgt.

3. Verfahren gemäß Anspruch 2, worin das Gewichtsverhältnis von Lösungsmittel zu Reaktand (einschließlich aller vorhandenen Isomere) 1:1 bis 2,5:1 beträgt.

4. Verfahren gemäß einem der Ansprüche 1 bis 3, worin das Nitrierungsmittel eine Mischung aus Salpeter- und Schwefelsäure ist, die 30 bis 45 % reine Salpetersäure enthält.

5. Verfahren gemäß Anspruch 4, worin das Nitrierungsmittel zur Reaktionsmischung über einen Zeitraum von ca. 30 min bis 15 h hinzugegeben wird.

6. Verfahren gemäß einem der Ansprüche 1 bis 5, worin in der Verbindung der allgemeinen Formel (I) R² Chlor ist und R³ Trifluormethyl ist.

7. Verfahren gemäß Anspruch 6, worin die Verbindung der allgemeinen Formel (I) 5-(2-Chlor-α,α,α-trifluor-4-tolyloxy)-2-nitrobenzoesäure (Acifluorfen) oder 5-(2-Chlor-α,α,α-trifluor-4-tolyloxy)-N-methansulfonyl-2-nitrobenzamid (Fomesafen) ist.

8. Verfahren gemäß Anspruch 7, worin die Verbindung der allgemeinen Formel (I) Acifluorfen ist und welches zusätzlich die Schritte der Konvertierung des Acifluorfens zu seinem Säurechlorid und die Behandlung des Säurechlorids mit Methansulfonamid zum Erhalt von Fomesafen umfaßt.

## Revendications

1. Procédé de préparation d'un composé de formule générale I 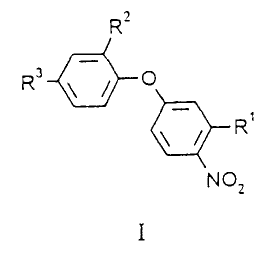 dans laquelle
- R¹ représente un atome d'hydrogène ou un groupe alkyle C₁-C₆, alkényle C₂-C₆ ou alkynyle C₂-C₆ (l'un quelconque d'entre eux pouvant être éventuellement substitué par un ou plusieurs substituants choisis parmi les halogènes et OH) ou COOR⁴, COR⁶, CONR⁴R⁵ ou CONHSO₂R⁴ ;
R⁴ et R⁵ représentent chacun indépendamment l'hydrogène ou un groupe alkyle C₁-C₄ éventuellement substitué par un ou plusieurs atomes d'halogène ;
R⁶ représente un atome d'halogène ou un groupe R⁴ ;
- R² représente un atome d'hydrogène ou un halogène ;
- R³ représente un groupe alkyle C₁-C₄, alkényle C₂-C₄ ou alkynyle C₂-C₄, (l'un quelconque d'entre eux pouvant être éventuellement substitué par un ou plusieurs atomes d'halogène) ou un halogène ;
le procédé comprenant la réaction d'un composé de formule générale II 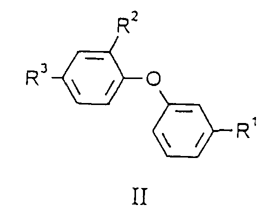 dans laquelle R¹, R² et R³ sont tels que définis pour la formule générale I ;
avec un agent nitrant comprenant de l'acide nitrique ou un mélange d'acide nitrique et d'acide sulfurique en présence d'un solvant organique et en présence d'anhydride acétique, où le rapport molaire entre l'anhydride acétique et le composé de formule générale II est compris entre environ 1/1 et 3/1,
caractérisé en ce que le solvant organique est le tétrachloroéthylène (perklone).

2. Procédé selon la revendication 1, dans lequel le rapport pondéral solvant/réactant (y compris tous les isomères présents) n'est pas supérieur à 4,25/1.

3. Procédé selon la revendication 2, dans lequel le rapport pondéral solvant/réactant (y compris tous les isomères présents) est compris entre 1/1 et 2,5/1.

4. Procédé selon l'une quelconque des revendications 1 à 3, dans lequel l'agent nitrant est un mélange d'acide nitrique et d'acide sulfurique contenant 30 à 45 % d'acide nitrique pur.

5. Procédé selon la revendication 4, dans lequel l'agent nitrant est additionné au mélange réactionnel sur une période d'environ 30 minutes à 15 heures.

6. Procédé selon l'une quelconque des revendications 1 à 5, dans lequel, dans le composé de formule générale I, R² représente le groupe chloro et R³ représente le groupe trifluorométhyle.

7. Procédé selon la revendication 6, dans lequel le composé de formule générale I est l'acide 5-(2-chloro-α,α,α-trifluoro-4-tolyloxy)-2-nitrobenzoïque (Acifluorfen) ou le 5-(2-chloro-α,α,α-trifluoro-4-tolyloxy)-N-méthanesulfonyl-2-nitrobenzamide (Fomesafen).

8. Procédé selon la revendication 7, dans lequel le composé de formule générale I est l'Acifluorfen et qui comprend aussi les étapes de conversion de l'Acifluorfen en son chlorure d'acide et de traitement du chlorure d'acide avec le méthane sulfonamide pour donner le Fomesafen.
